# EUROPEAN PATENT APPLICATION

(11) **EP 2 058 406 A2**
(43) Date of publication of application: **13.05.2009**
(21) Application number: 08019382.4
(22) Date of filing: 05.11.2008
(51) Int. Cl.: C12Q 1/68

(54) **RNA detection method**

(30) Priority: 06.11.2007 JP 2007288214
(71) Applicant: Fujifilm Corporation, Tokyo 106-0031 (JP)
(72) Inventor: Miyoshi, Hayato, Ashigarakami-gun Kanagawa 258-8577 (JP); Iwaki, Yoshihide, Ashigarakami-gun Kanagawa 258-8577 (JP); Mori, Toshihiro, Ashigarakami-gun Kanagawa 258-8577 (JP)
(74) Representative: HOFFMANN EITLE

(57) **Abstract**

It is an object of the present invention to provide a method for rapid, convenient, and highly sensitive detection of trace RNA wherein a risk of contamination is low. The present invention provides a method for amplification of nucleic acid which comprises the steps of: (i) allowing a reverse transcriptase to act on RNA so as to produce a nucleic acid fragment; and (ii) performing substantially isothermal incubation of a reaction solution containing at least one type of deoxynucleotide triphosphate, at least one type of DNA polymerase having strand displacement activity, a divalent cation, a surfactant accounting for at least 0.01% of the solution, at least two types of oligonucleotide primers, and the nucleic acid fragment as a template obtained in the step (i) so as to perform a polymerase reaction that is initiated from the 3' ends of the primers and thus amplify the nucleic acid fragment.

## Description

### Technical Field

The present invention relates to an RNA detection method. More specifically, the present invention relates to a method for rapid, convenient, and highly sensitive detection of trace RNA. It also relates to an RNA detection method with a low risk of contamination.

### Background Art

In molecular biological research and clinical diagnosis, nucleic acid amplification methods are used as essential techniques for detecting trace nucleic acid in a wide range of fields. At present, nucleic acid amplification methods are actively used as methods for detecting trace RNA. According to the RT-PCR method that is generally used as an RNA detection method, a reverse transcriptase is first allowed to act on RNA such that DNA complementary to the RNA is produced. Then, the thus produced DNA is amplified by a PCR (polymerase chain reaction) method using primers specific to a target gene. For amplification of a target nucleic acid sequence, the PCR method comprises the three steps of: denaturing (denaturation step) double-stranded DNA as a template into single-stranded DNAs; annealing (annealing step) primers to the single-stranded DNAs; and elongating (elongation step) complementary strands using the primers as origins. According to a general PCR method, the denaturation step, the annealing step, and the elongation step are each performed at different temperatures using a thermal cycler. However, implementation of nucleic acid amplification reactions at three different temperatures is problematic in that temperature control is complicated and time loss increases in proportion to the number of cycles.

Hence, nucleic acid amplification methods that can be performed under isothermal conditions have been developed. Examples of such methods include RCA (Rolling Circle Amplification: Proc. Natl. Acad. Sci, vol. 92, 4641-4645 (1995)), ICAN (Isothermal and Chimeric primer-initiated Amplification of Nucleic acids), LAMP (Loop-Mediated Isothermal Amplification of DNA; Bio Industry, vol. 18, No. 2 (2001)), NASBA (Nucleic acid Sequence-based Amplification method; Nature, 350, 91 - (1991)), and TMA (Transcription mediated amplification method; J. Clin Microbiol. Vol. 31, 3270 - (1993)).

SDA method (JP Patent Publication (Kokai) No. 5-130870 A (1993)) is a cycling assay method using exonuclease, which is a method for amplifying a target site of a target nucleic acid fragment using a polymerase elongation reaction. This method comprises performing a polymerase elongation reaction using primers (as origins) that have specifically hybridized to target sites of target nucleic acid fragments, while causing 5'→3'exonuclease to act thereon, so as to degrade the primers from the opposite directions. New primers undergo hybridization instead of the degraded primers, so that another elongation reaction proceeds again with the use of DNA polymerase. Such an elongation reaction with the use of polymerase and such a degradation reaction with the use of exonuclease by which the strand that has been elongated is removed are repeated periodically in order. Here, the elongation reaction with the use of polymerase and the degradation reaction with the use of exonuclease can be implemented under isothermal conditions. However, the use of exonuclease in addition to polymerase is required, and thus the method is expensive and the design of primers should be improved.

LAMP method is a method for amplifying target sites of a target nucleic acid fragment that has been developed in recent years. This method is a method for amplifying target sites of a target nucleic acid fragment as special structures under isothermal conditions through the use of at least four types of primers that complementarily recognize at least six specific sites of a target nucleic acid fragment and strand-displacement-type Bst DNA polymerase lacking 5'→3' nuclease activity and catalyzing an elongation reaction while liberating double-stranded DNA on the template in the form of single-stranded DNAs. However, the method requires the use of at least four types of primers that recognize six specific sites, so that the design of primers is very difficult.

ICAN method is a method for amplifying target sites of a target nucleic acid fragment that has been developed in recent years. The ICAN method is an isothermal gene amplification method using RNA-DNA chimeric primers, DNA polymerase having strand displacement activity and template exchange activity, and RNaseH. After chimeric primers bind to a template, a complementary strand is synthesized by DNA polymerase. Subsequently, RNaseH cleaves RNA portions derived from the chimeric primers and then an elongation reaction accompanied by a strand displacement reaction and a template exchange reaction takes place repeatedly from the cleaved sites, so that the gene amplification is performed. However, this method also requires the use of special primers that are chimeric primers and thus the design of such primers is very difficult.

JP Patent Publication (Kohyo) No. 11-509406 A (1999) discloses an amplification method by which, in the presence of DNA polymerase capable of strand displacement, DNA within a target region is amplified by an isothermal reaction using at least a set of oligonucleotide primers. However, in the case of the method disclosed in JP Patent Publication (Kohyo) No. 11-509406 A (1999), a relatively long period of reaction time is necessary, which is problematic. That is, as with the PCR method, the development of nucleic acid amplification methods that can be readily carried out under isothermal conditions with the simple design of primers has been awaited.

JP Patent Publication (Kokai) No. 2002-233379 A discloses an amplification method by which, in the presence of DNA polymerase capable of strand displacement, DNA within a target region is amplified by an isothermal reaction using at least a set of oligonucleotide primers. However, in the case of the method disclosed in JP Patent Publication (Kokai) No. 2002-233379 A, nonspecific amplified products are generated to a significant degree, which is problematic.

### SUMMARY OF THE INVENTION

It is an object of the present invention to provide a method for rapid, convenient, and highly sensitive detection of trace RNA wherein a risk of contamination is low. It is another object of the present invention to provide a method for detection of RNA with simpler design of primers.

As a result of intensive studies to solve the above objects, the present inventors have found that nucleic acid can be efficiently amplified within a short time by allowing a reverse transcriptase to act on RNA so as to produce DNA complementary to the RNA, adding the DNA to a reaction solution containing deoxynucleotide triphosphate, DNA polymerase having strand displacement activity, a divalent cation, a surfactant, and oligonucleotide primers, and performing substantially isothermal incubation so as to perform a polymerase reaction that is initiated from the 3' ends of the primers. Further, the oligonucleotide primer used in the present invention is characterized in that it does not have such a complicated structure as those used in the conventional isothermal amplification methods. Namely, the oligonucleotide primer used in the present invention dose not require a structure which forms a chimera structure used in ICAN method or a loop structure used in LAMP method.

Further, the present inventors have found a composition of a reaction solution in which both the reverse transcriptase and DNA polymerase having strand displacement activity can exhibit their activities. Consequently, they succeeded in sequentially carrying out the following steps in a single reaction vessel: a step of producing DNA complementary to RNA with the use of the RNA as a template; a step of carrying out nucleic acid amplification with the use of the thus produced DNA as a template; and a step of detecting an amplified product. This has led to the completion of the present invention.

The present invention provides a method for amplification of nucleic acid which comprises the steps of:
(i) allowing a reverse transcriptase to act on RNA so as to produce a nucleic acid fragment; and
(ii) performing substantially isothermal incubation of a reaction solution containing at least one type of deoxynucleotide triphosphate, at least one type of DNA polymerase having strand displacement activity, a divalent cation, a surfactant accounting for at least 0.01 % of the solution, at least two types of oligonucleotide primers, and the nucleic acid fragment as a template obtained in the step (i) so as to perform a polymerase reaction that is initiated from the 3' ends of the primers and thus amplify the nucleic acid fragment.

Preferably, the step (i) of allowing a reverse transcriptase to act on RNA so as to produce a nucleic acid fragment and the step (ii) of amplifying the nucleic acid fragment are sequentially carried out in a single reaction vessel.

Preferably, the reverse transcriptase is a reverse transcriptase selected from the group consisting of avian myeloblastosis virus-derived AMV RTase, moloney murine leukemia virus-derived MMLV RTase, SuperScript II that is an RNaseH activity-deficient mutant of moloney murine leukemia virus-derived reverse transcriptase, and rous associated virus 2-derived RAV-2 RTase.

Preferably, the surfactant is a nonionic surfactant.

Preferably, the HLB value of the nonionic surfactant is 12 or more.

Preferably, the HLB value of the nonionic surfactant is 14 or more.

Preferably, the nonionic surfactant is selected from among a polyoxyethylene sorbitan fatty acid ester-based surfactant, and a polyoxyethylene alkyl ether-based surfactant.

Preferably, the polyoxyethylene sorbitan fatty acid ester-based nonionic surfactant is polyoxyethylene sorbitan mono fatty acid ester.

Preferably, the nonionic surfactant is represented by the following formula: 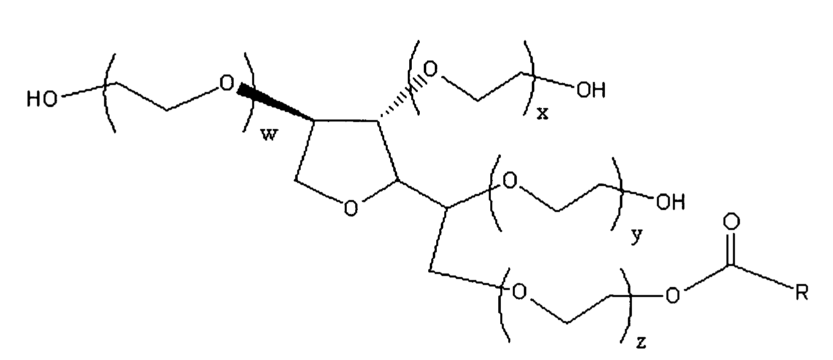 wherein x + y + z + w = 20, R is an alkyl group having a carbon number of 12 to 18.

Preferably, the polyoxyethylene sorbitan fatty acid ester-based nonionic surfactant is at least one which is selected from polyoxyethylene(20) sorbitan monolaurate, polyoxyethylene(20) sorbitan monopalmitate, polyoxyethylene(20) sorbitan monostearate, and polyoxyethylene(20) sorbitan monooleate.

Preferably, the reaction solution further contains a melting temperature adjusting agent.

Preferably, the melting temperature adjusting agent is dimethyl sulfoxide, betaine, formamide, or glycerol, or a mixture of two or more types thereof.

Preferably, a reaction solution contains each deoxynucleotide triphosphate of 1.0 mM or more.

Preferably, the reaction solution contains oligonucleotide primer of 1µM or more.

Preferably, the oligonucleotide primers are substantially complementary to portions of the template nucleic acid fragment obtained in the step (i).

Preferably, only the 3'-terminal region of the oligonucleotide primers is substantially complementary to portions of the template nucleic acid fragment obtained in the step (i).

Preferably, the oligonucleotide primers are substantially complementary to only consecutive 1 site of the template nucleic acid fragment obtained in the step (i).

Preferably, at least one type of polymerase having strand displacement activity is a polymerase selected from the group consisting of *Bacillus stearothermophilus-derived* 5'→3' exonuclease-deficient Bst. DNA polymerase, *Bacillus caldotenax-derived* 5'→3'exonuclease-deficient Bca DNA polymerase, *Thermococcus litoralis-derived* 5'→3' exonuclease-deficient Vent. DNA polymerase, and *Alicyclobacillus acidocaldarius*-derived DNA polymerase.

Preferably, the reaction solution is incubated substantially isothermally at a temperature of 50°C or more.

Preferably, the time for the substantially isothermal incubation in the step (ii) is within 60 minutes.

The present invention further provides a method for detecting a target RNA which comprises performing the method for amplification of nucleic acid as mentioned above.

Preferably in the method for detecting a target RNA as mentioned above, the step (ii) comprises the following steps of:
(1) substantially isothermally incubating a reaction solution containing at least one type of deoxynucleotide triphosphate, at least one type of DNA polymerase having strand displacement activity, a divalent cation, at least one type of nonionic surfactant, at least two types of oligonucleotide primer containing a mutation site, and a nucleic acid fragment containing a reverse transcript of the target RNA as a template; and
(2) determining the presence or the absence of a mutation based on whether or not a nucleic acid amplification reaction takes place by a polymerase reaction that is initiated from the 3' end of the primer.

According to the present invention, the present inventors have found that nucleic acid can be efficiently amplified within a short time by allowing a reverse transcriptase to act on RNA so as to produce DNA complementary to the RNA, adding the DNA to a reaction solution containing deoxynucleotide triphosphate, DNA polymerase having strand displacement avtivity, a divalent cation, a surfactant, and oligonucleotide primers, and performing substantially isothermal incubation so as to induce a polymerase reaction that is initiated from the 3' ends of the primers.

In addition, a step of carrying out nucleic acid amplification can be performed isothermally. Thus, there is no need to increase or decrease temperature in a periodic manner. Accordingly, it has become possible to carry out the above steps in a simple apparatus.

Further, the oligonucleotide primer used in the present invention is characterized in that it does not have such a complicated structure as those used in the conventional isothermal amplification methods. Namely, the oligonucleotide primer used in the present invention does not require a structure which forms a chimera structure used in ICAN method or a loop structure used in LAMP method.

Further, the above steps can be carried out in a single reaction vessel. In such case, detection can be carried out in a more convenient and rapid manner. Furthermore, an RNA detection method with a low risk of contamination can be realized. The present invention originally involves a nucleic acid amplification reaction and thus it also involves a highly sensitive RNA detection method.

### BRIEF DESCRIPTION OF THE DRAWINGS

Fig. 1 shows in detail the positional relationship of the primers used in the Examples and the reverse-transcribed DNA and its complementary strand of β-actin mRNA.
Fig. 2 shows results of fluorescence detection of an amplified product obtained as a result of the amplification reaction of the present invention.
Fig. 3 shows results of fluorescence detection of an amplified product obtained as a result of the amplification reaction of the present invention.
Fig. 4 shows results of fluorescence detection of an amplified product obtained as a result of the amplification reaction used in the Comparative Example.

### PREFERRED EMBODIMENT OF THE INVENTION

The present invention will be further described in detail as follows.

The RNA detection method of the present invention is characterized by the following steps of: allowing a reverse transcriptase to act on RNA so as to produce DNA complementary to the RNA; adding the DNA to a reaction solution containing deoxynucleotide triphosphate, DNA polymerase having strand displacement activity, a divalent cation, a surfactant, and oligonucleotide primers; and performing substantially isothermal incubation so as to induce a polymerase reaction that is initiated from the 3' ends of the primers.

Further, the above steps can be carried out in a single reaction vessel.

Hereinafter, ingredients that are used in the present invention will be explained.

### (1) Deoxynucleotide triphosphate

Deoxynucleotide triphosphate is used as a substrate for an elongation reaction. Specifically, a mixture of dATP, dCTP, dGTP, and dTTP is preferably used. Deoxynucleotide triphosphate to be used herein may contain a dNTP analog (e.g., 7-deaza-dGTP).

Furthermore, deoxynucleotide triphosphate (dATP, dCTP, dGTP, or dTTP mixture) is at a final concentration ranging from 0.1 mM to 3.0 mM, preferably 0.75 mM to 3.0 mM, further preferably 1.0 mM to 2.0 mM, and particularly preferably 1.0 mM to 1.5 mM.

### (2) Reverse transcriptase

In the present invention, a reverse transcriptase is used. Examples of a reverse transcriptase that can be used are not particularly limited, as long as it has activity of synthesizing DNA with the use of a target RNA as a template. Examples of the reverse transcriptase include avian myeloblastosis virus-derived reverse transcriptase (AMV RTase), moloney murine leukemia virus-derived reverse transcriptase (MMLV RTase), and rous associated virus 2 reverse transcriptase (RAV-2 RTase). Furthermore, strand displacement-type DNA polymerase that has reverse transcription activity can also be used.

### (3) Oligonucleotide primer for reverse transcription

A primer to be used for a reverse transcription reaction may be a primer having a nucleotide sequence complementary to a specific template RNA, an oligo dT primer, or a primer having a random sequence. The length of a primer for reverse transcription preferably ranges from approximately 3 to 100 nucleotides and further preferably ranges from 6 to 50 nucleotides.

### (4) DNA polymerase

In the present invention, DNA polymerase is used. The DNA polymerase that can be used is preferably a polymerase having strand displacement activity. In the description, "strand displacement activity" refers to activity by which strand displacement can be performed; that is, when DNA replication is performed based on a template nucleic acid sequence, strand displacement proceeds by replacement of DNA strands, so as to liberate a complementary strand that has annealed to the template strand. Specific examples of polymerase having strand displacement activity include, but are not limited to, *Bacillus stearothermophilus*-derived 5'→3' exonuclease-deficient Bst. DNA polymerase, *Bacillus caldotenax*-derived 5'→3' exonuclease-deficient Bca DNA polymerase, *Thermococcus litoralis*-derived 5'→3' exonuclease-deficient Vent. DNA polymerase, and *Alicyclobacillus acidocaldarius*-derived DNA polymerase. Such polymerase having strand displacement activity may be derived from nature or may be a genetically engineered recombinant protein.

### (5) Divalent cation

In the present invention, divalent cations are used in response to metal requirements and the like regarding enzymes to be used herein. As divalent cations, magnesium salts or other metal salts can be used. For example, magnesium chloride, magnesium acetate, and magnesium sulfate can be used. Such a divalent cation is at a final concentration preferably ranging from 1 mM to 20 mM and further preferably ranging from 2 mM to 10 mM.

### (6) Surfactant

In the present invention, a surfactant is added to a reaction solution. An advantagenous effect of the present invention; that is, prevention of nonspecific nucleic acid amplification, is achieved via the use of a surfactant. Types of such surfactant that can be used in the present invention are not particularly limited, and may include the following:
anionic surfactants such as alkylbenzene sulfonate, lauryl sulfate (SDS), octyl sulfosuccinate, and stearic acid soap;
nonionic surfactants such as sorbitan fatty acid ester, POE sorbitan fatty acid ester (e.g., Tween), POE alkyl ether (e.g., Brij), POE alkyl phenyl ether (e.g., Triton), nonylphenol, lauryl alcohol, polyethylene glycol, polyoxyethylene-polyoxypropylene block polymer, POE alkyl amine, and POE fatty acid bisphenyl ether;
cationic surfactants such as cetylpyridium chloride, lauryl dimethylbenzyl ammonium chloride, and stearyltrimethylammonium chloride; and
ampholytic surfactants such as alkyldimethylamine oxide and alkylcarboxybetaine.

The dose of such a surfactant is not particularly limited, as long as the effects of the present invention can be achieved and is preferably 0.01% or more, more preferably 0.05% or more, and more preferably 0.1% or more. The upper limit of the dose of such a surfactant is not particularly limited and is generally 10% or less, preferably 5% or less, and more preferably 1 % or less.

Among the above surfactants, nonionic surfactants are preferably used. Among the nonionic surfactants, highly hydrophilic surfactants are preferred. The HLB value is preferably 12 or more, and further preferably 14 or more. Preferably, the upper limit of HLB is 20. Preferably, the value of HLB is 17 or less. More preferably, the value of HLB is 14 to 17. The surfactant is preferably selected from a polyoxyethylene sorbitan fatty acid ester-based surfactant, and a polyoxyethylene alkyl ether-based surfactant. Among the polyoxyethylene sorbitan fatty acid ester, polyoxyethylene sorbitan mono fatty acid ester is preferred. Preferably the compound represented by the following formula can be used: 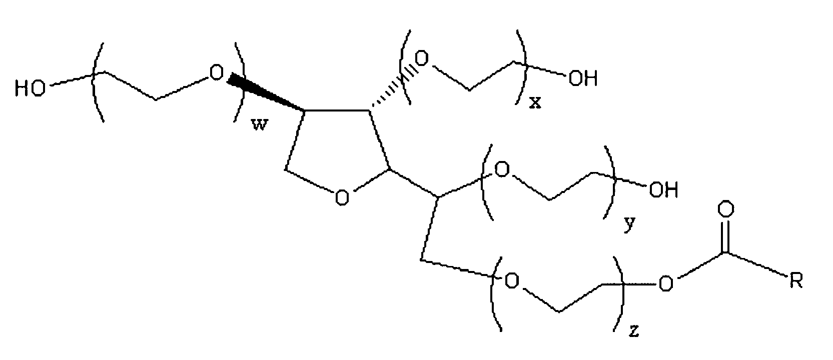 wherein x + y + z + w = 20, R is an alkyl group having a carbon number of 12 to 18.

The position of the alkyl group is not particularly limited, and the compound of the following structure can be preferably used. 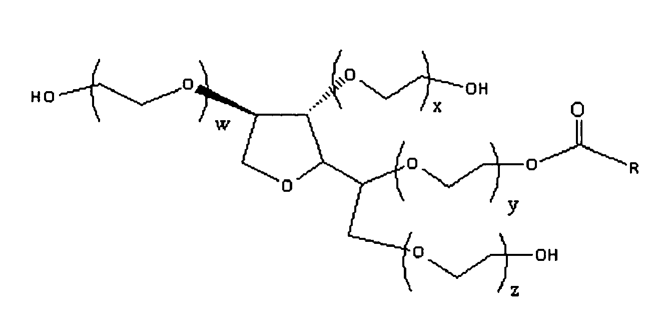 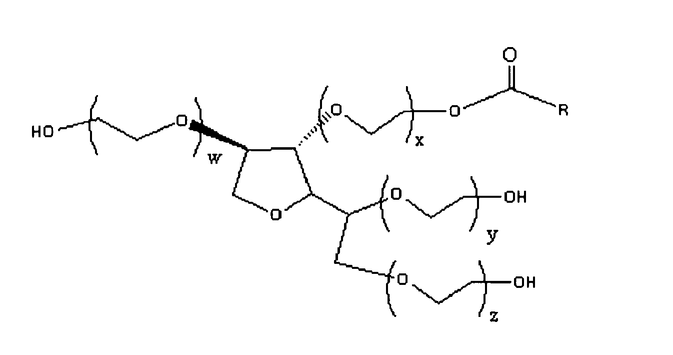 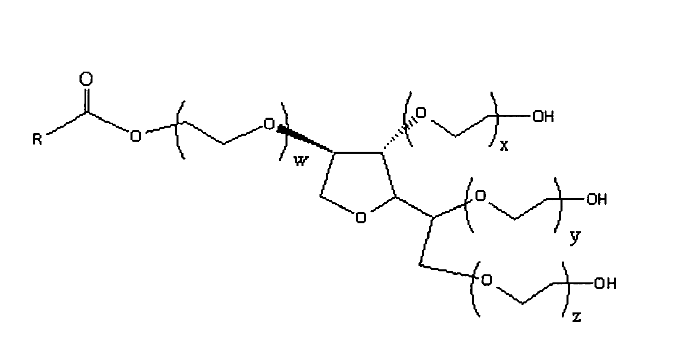 wherein x + y + z + w = 20, R is an alkyl group having a carbon number of 12 to 18.

Specific examples of such surfactants may include polyoxyethylene(20) sorbitan monolaurate, polyoxyethylene(20) sorbitan monopalmitate, polyoxyethylene(20) sorbitan monostearate, and polyoxyethylene(20) sorbitan monooleate (trade name: Tween 20, Tween 40, Tween 60, Tween 80, and the like). The dose of such surfactant is not particularly limited, and may be preferably 0.01 % or more, more preferably 0.05% or more, and more preferably 0.1% or more.

### (7) Oligonucleotide primer

The oligonucleotide primer to be used in the nucleic acid amplification reaction of the present invention has a nucleotide sequence substantially complementary to template DNA and has the 3' end from which DNA strand elongation is possible. Such oligonucleotide primer has a nucleotide sequence substantially complementary to template DNA, so that it can anneal to the template DNA. As an oligonucleotide primer to be used in the present invention, an oligonucleotide primer composed of a deoxyribonucleotide or a ribonucleotide can be used. Furthermore, an oligonucleotide primer containing a modified ribonucleotide or a modified deoxyribonucleotide may also be used herein.

For the aforementioned oligonucleotide primer, no complicated design such as those employed for conventional isothermal amplification reactions is required. An important feature of the present invention is resides in that isothermal amplification reactions can be carried out by using at least one set of primers which are used in the general PCR. Especially, these primers do not have a structure which forms a loop structure wherein 5' terminal is complementary to the region which was elongated from the 3'terminal as used in LAMP method. Namely, the consecutive region at 3'-terminal of the primer is complementary to the template nucleic acid. Further, the oligonucleotide primer has no complicated system where the primer is cleaved during the reaction and the cleaved 3' terminal serves as a synthesis origin, which is used in the SDA method or the ICAN method.

The length of an oligonucleotide primer is not particularly limited and generally ranges from approximately 10 to 100 nucleotides, preferably ranges from approximately 15 to 50 nucleotides, and further preferably ranges from approximately 15 to 40 nucleotides.

Oligonucleotide primers can be synthesized by the phosphoamidite method using a commercially available DNA synthesizer (e.g., Applied Biosystem Inc., DNA synthesizer 394).

The dose of an oligonucleotide primer is preferably 0.1 µM or more (for example, 0.1 µM to 100 µM), further preferably 1 µM or more (for example 1 µM to 50 µM), and particularly preferably 1.5 µM or more (for example, 1.5 µM to 10 µM) in a reaction solution.

### (8) Template RNA

In the present invention, template RNA may be any of mRNA, total RNA, rRNA, siRNA, virus genome RNA and the like. RNA that is prepared from a sample that may contain template RNA may also be used. A sample that may contain template RNA may also be directly used intact. Examples of the type of a sample containing template RNA are not particularly limited and include body fluids (e.g., whole blood, serum, urine, cerebrospinal fluid, seminal fluid, and saliva), tissues (e.g., cancer tissue), *in vivo* derived samples such as swab and cell culture products, RNA-containing samples such as viruses, bacteria, fungi, yeast, plants and animals, samples that may be contaminated with microorganisms (e.g., foods), or samples in an environment such as soil or waste water. When RNA is prepared from a sample described above, the preparation method therefor is not particularly limited. For example, methods known by persons skilled in the art can be used, including treatment using a surfactant, ultrasonication, purification using glass beads, and the like. Purification of RNA from such a sample can be performed by phenol extraction, chromatography, gel electrophoresis, density gradient centrifugation, or the like.

### (9) Pretreatment of template RNA

The template RNA in the present invention may be used after being subjected to pretreatment.

The reagent used for the pretreatment may contain, for example, a surfactant, an inhibitor of blood coagulation, a protease, or a lipase. The solution of the reagent may be acidic or alkaline.

The pretreatment may contain a step of heating at a high temperature (for example, 98°C) or a step of treatment with a denaturing agent. Further, the pretreatment may contain a step of rapidly cooling to 4°C or less after heating at a high temperature.

### (10) Melting temperature adjusting agent

A melting temperature adjusting agent can be added to a reaction solution in the present invention. Specific examples of such a melting temperature adjusting agent include dimethyl sulfoxide (DMSO), betaine, formamide or glycerol, tetraalkyl ammonium salt, and a mixture of two or more types thereof. The dose for melting temperature adjustment is not particularly limited. In the case of DMSO, formamide, or glycerol, a melting temperature adjusting agent can be generally contained accounting for 10% or less of a reaction solution.

Betaine or tetraalkyl ammonium salt can be added at a concentration ranging from approximately 0.2 M to 3.0 M, preferably approximately 0.5 M to 1.5 M.

### (11) Buffer component

A reaction solution in the present invention can contain a buffer component. Examples of such a buffer component that can be used herein include, but are not particularly limited to, bicin, tricine, hepes, tris, and phosphate (e.g., sodium phosphate and potassium phosphate). The final concentration of such a buffer component ranges from 5 mM to 100 mM and particularly preferably ranges from 10 mM to 50 mM. Regarding pH, such a buffer component having pH generally ranging from 6.0 to 9.0 and particularly preferably ranging from 7.0 to 9.0 can be used, depending on optimum pH for an enzyme to be used for an amplification reaction.

### (12) Fluorescent dye

The reaction solution used in the present invention may contain a fluorescent dye. Examples of a fluorescent dye may include, but are not particularly limited to, SYBR Green I.

Amplified products obtained by the nucleic acid amplification method of the present invention can be detected by methods known by persons skilled in the art. For example, according to gel electrophoresis, gel is stained with ethidium bromide and then reaction products of a specific size can be detected. As detection systems for detection of amplified products, fluorescence polarization, immunoassay, fluorescent energy transfer, enzyme labels (e.g., peroxidase and alkaline phosphatase), fluorescent labels (e.g., fluorescein and rhodamine), chemiluminescence, bioluminescence, or the like can be used. Also, Taqman probes and Molecular Beacon can be used for detection. Amplified products can also be detected using a labeled nucleotide labeled with biotin or the like. In such a case, biotin in an amplified product can be detected using fluorescence labeled avidin, enzyme-labeled avidin, or the like. In addition, amplified products can be detected by an electrode with the use of a redox intercalator known to persons skilled in the art. Alternatively, an SPR may be used to detect amplified products.

Also, nucleic acid amplification can be detected by detecting magnesium pyrophosphate. In such a case, detection can be carried out by a method involving detection based on turbidity or the like, which is known to persons skilled in the art.

The present invention will be specifically described in the following examples. However, the examples are not intended to limit the present invention.

### EXAMPLES

### <Example 1> Detection of β-actin mRNA in total RNA

### (1) Production of DNA complementary to template RNA by reverse transcription reaction

Pure water was added to Human Liver Total RNA (0.1 µg, Clonetech), Random 6mer Primer (100 pmol, TaKaRa), and dNTP Mixture (10 nmol) to a final volume of 13 µl, followed by heating at 65°C for 5 minutes.

A 5-fold-concentrated reverse transcription buffer (4 µl) and 100 mM dithiothreitol (2 µl) were added to the reaction solution, followed by heating at 42°C for 2 minutes.

SuperScript II (1 µl, Invitrogen) was added to the reaction solution, followed by heating at 25°C for 10 minutes, 42°C for 50 minutes, and 70°C for 10 minutes.

### (2) Nucleic acid amplification reaction with the use of DNA produced in (1) as a template

The following primers were used to carry out sequence-specific nucleic acid amplification.

### <Primers>

Primers were designed using the reverse transcribed DNA (and its complementary sequence) of β-actin mRNA as a target. Each primer sequence is as shown below.
Primer (1) (Forward):
5'-GGGCATGGGTCAGAAGGATT-3' (SEQ ID NO: 1)
Primer (2) (Reverse):
5'-CCTCGTCGCCCACATAG-3' (SEQ ID NO: 2)

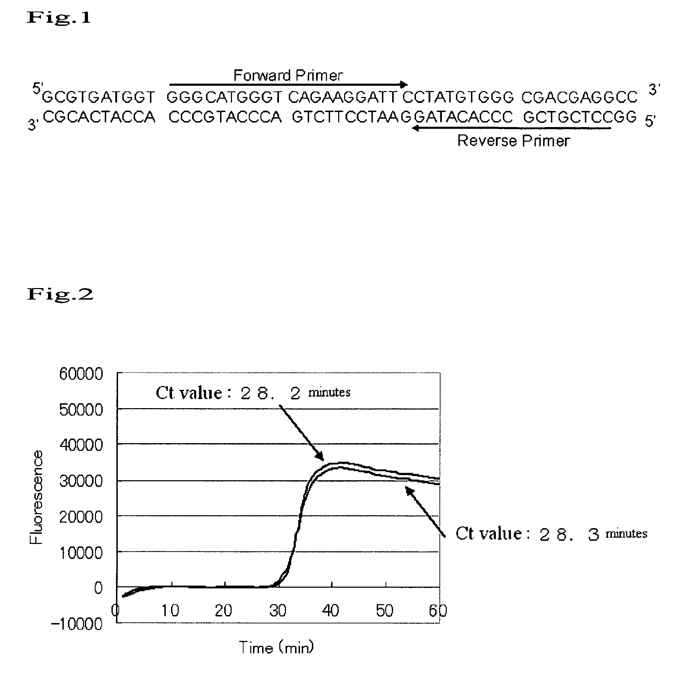

Fig. 1 shows in detail the positional relationship of the primers and the reverse-transcribed DNA and its complementary strand of β-actin mRNA.

The amplification reaction was performed with the composition of a reaction solution shown below at 60°C for 60 minutes. Bst. DNA polymerase (NEB (New England Biolabs)) was used as an enzyme.

### <Composition of reaction solution>

| | |
|---|---|
| 10 × Bst Buffer | 2.5 µL |
| MgSO₄ (100 mM) | 1.5 µL |
| Tween20 (10% (v/v)) | 0.25 µL |
| DMSO | 1.25 µL |
| dNTP (25 mM each) | 1.4 µL |
| SYBR Green I (2000-fold diluted) | 0.5 µL |
| 50 µM primer (1) | 1.6 µL |
| 50 µM primer (2) | 1.6 µL |
| Bst. Polymerase | 1.0 µL |
| Solution obtained in (1) | 1.0 µL |
| Purified water | 12.4 µL |
| | 25.0 µL |

### (3) Detection of amplified product

Products of the amplification reaction in (2) above were subjected to fluorescence detection using a real-time fluorescence detection apparatus (Mx3000p, Stratagene). Fig. 2 shows the results.

It was shown that nucleic acid amplification took place in the case of samples derived from RNA specimen. Here, the time (Ct value) that it took for an amount of fluorescence to reach 250 in the above graph was calculated using Mx3000p analysis software. The results were 28.2 minutes and 28.3 minutes (experimentation: n = 2).

### <Example 2> RNA detection in a single reaction vessel

### (1) Reverse transcription reaction and nucleic acid amplification reaction

### <Template RNA>

Human Liver Total RNA (0.1 µg, Clonetech) was used as a template RNA.

The following primers were used to carry out sequence-specific nucleic acid amplification.

### <Primers>

Primers were designed using the reverse transcribed DNA (and its complementary sequence) of β-actin mRNA as a target. Each primer sequence is as shown below.
Primer (1) (Forward):
5'-GGGCATGGGTCAGAAGGATT-3' (SEQ ID NO: 1)
Primer (2) (Reverse):
5'-CCTCGTCGCCCACATAG-3' (SEQ ID NO: 2)

The amplification reaction was performed with the composition of a reaction solution shown below at 25°C for 10 minutes, 42°C for 50 minutes, and 60°C for 60 minutes. Bst. DNA polymerase (NEB (New England Biolabs)) was used as an enzyme.

### <Composition of reaction solution>

| | |
|---|---|
| Template RNA (0.1 µg/µL) | 1.0 µL |
| Random 6mer Primer (100 µM) | 1.0 µL |
| Dithiothreitol (0.1 µM) | 2.0 µL |
| 10 × Bst Buffer | 2.5 µL |
| MgSO₄ (100 mM) | 1.5 µL |
| Tween20 (10% (v/v)) | 0.25 µL |
| DMSO | 1.25 µL |
| dNTP (25 mM each) | 1.8 µL |
| SYBR Green I (2000-fold diluted) | 0.5 µL |
| 50 µM primer (1) | 1.6 µL |
| 50 µM primer (2) | 1.6 µL |
| RTase (SuperScript II) | 1.0 µL |
| Bst. Polymerase | 1.0 µL |
| Purified water | 17.0 µL |
| | 25.0 µL |

### (2) Detection of amplified product

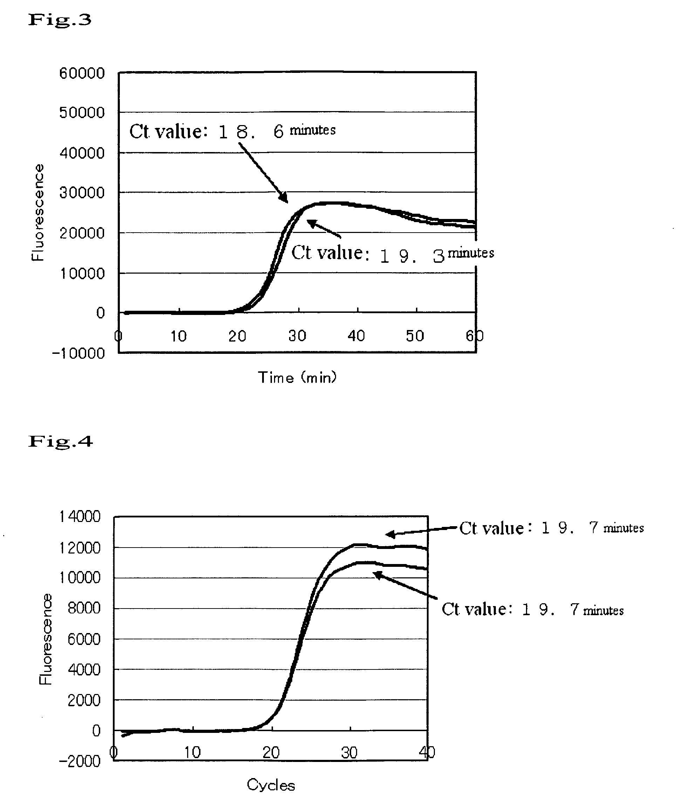

Products of the amplification reaction in (1) above were subjected to fluorescence detection using a real-time fluorescence detection apparatus (Mx3000p, Stratagene). Fig. 3 shows the results.

It was shown that nucleic acid amplification took place in the case of RNA sample. Here, the time (Ct value) that it took for an amount of fluorescence to reach 250 in the above graph was calculated using Mx3000p analysis software. The results were 18.6 minutes and 19.3 minutes (experimentation: n = 2). The Ct value takes "the time when the reaction was initiated at 60°C" as "0 minute".

As compared with Example 1, the time for detection could be shortened by about 10 minutes.

### <Comparative Example> Detection of β-actin mRNA in total RNA by the PCR method

### (1) Production of DNA complementary to template RNA by reverse transcription reaction

Pure water was added to Human Liver Total RNA (0.1 µg, Clonetech), Random 6mer Primer (100 pmol, TaKaRa), and dNTP Mixture (10 nmol) to a final volume of 13 µl, followed by heating at 65°C for 5 minutes.

A 5-fold-concentrated reverse transcription buffer (4 µl) and 100 mM dithiothreitol (2 µl) were added to the reaction solution, followed by heating at 42°C for 2 minutes.

SuperScript II (1 µl, Invitrogen) was added to the reaction solution, followed by heating at 25°C for 10 minutes, 42°C for 50 minutes, and 70°C for 10 minutes.

### (2) PCR with the use of DNA produced in (1) above as a template

The following primers were used to carry out sequence-specific nucleic acid amplification.

### <Primers>

Primers were designed using the reverse transcribed DNA (and its complementary sequence) of β-actin mRNA as a target. Each primer sequence is as shown below.
Primer (1) (Forward):
5'-GGGCATGGGTCAGAAGGATT-3' (SEQ ID NO: 1)
Primer (2) (Reverse):
5'-CCTCGTCGCCCACATAG-3' (SEQ ID NO: 2)

PCR was carried out with a reaction solution having the following composition.

### <Composition of reaction solution>

| | |
|---|---|
| 10 × PCR Buffer | 10.0 µL |
| 10 mM dNTP each | 4.0 µL |
| 50 µM primer (1) | 0.25 µL |
| 50 µM primer (2) | 0.25 µL |
| SYBR Green I (1000-fold diluted) | 0.5 µL |
| Taq Polymerase | 0.5 µL |
| Purified water | 37.5 µL |
| | 50.0 µL |

PCR was carried out with the following temperature cycle.

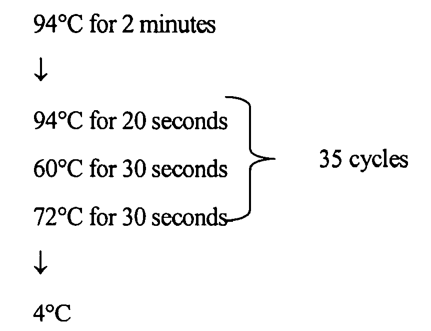

When Mx3000p is used as the real-time fluorescence detection apparatus, 1 cycle of this temperature cycle requires 2.1 minutes.

### (3) Detection of amplified product

Products of the amplification reaction in (2) above were subjected to fluorescence detection using a real-time fluorescence detection apparatus (Mx3000p, Stratagene). Fig. 4 shows the results.

It was shown that nucleic acid amplification took place in the case of samples derived from RNA specimen. Here, the number of cycles (Ct value) for an amount of fluorescence to reach 250 in the above graph was calculated using Mx3000p analysis software. The results were both 19.7 cycles (experimentation: n = 2), which corresponds to 41.4 minutes.

As compared with Example 1, the time for detection was increased by about 13 minutes. As compared with Example 2, the time for detection was increased by about 22 minutes.

## Claims

1. A method for amplification of nucleic acid which comprises the steps of:
(i) allowing a reverse transcriptase to act on RNA so as to produce a nucleic acid fragment; and
(ii) performing substantially isothermal incubation of a reaction solution containing at least one type of deoxynucleotide triphosphate, at least one type of DNA polymerase having strand displacement activity, a divalent cation, a surfactant accounting for at least 0.01 % of the solution, at least two types of oligonucleotide primers, and the nucleic acid fragment as a template obtained in the step (i) so as to perform a polymerase reaction that is initiated from the 3' ends of the primers and thus amplify the nucleic acid fragment.

2. The method of claim 1, wherein the step (i) of allowing a reverse transcriptase to act on RNA so as to produce a nucleic acid fragment and the step (ii) of amplifying the nucleic acid fragment are sequentially carried out in a single reaction vessel.

3. The method of claim 1, wherein the reverse transcriptase is a reverse transcriptase selected from the group consisting of avian myeloblastosis virus-derived AMV RTase, moloney murine leukemia virus-derived MMLV RTase, SuperScript II that is an RNaseH activity-deficient mutant of moloney murine leukemia virus-derived reverse transcriptase, and rous associated virus 2-derived RAV-2 RTase.

4. The method of claim 1, wherein the surfactant is a nonionic surfactant.

5. The method of claim 4 wherein the HLB value of the nonionic surfactant is 12 or more.

6. The method of claim 4, wherein the nonionic surfactant is selected from among a polyoxyethylene sorbitan fatty acid ester-based surfactant, and a polyoxyethylene alkyl ether-based surfactant.

7. The method of claim 4 wherein the nonionic surfactant is represented by the following formula: 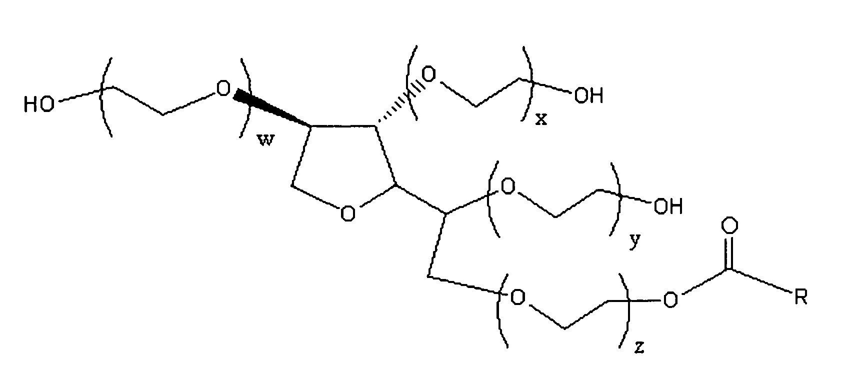 wherein x + y + z + w = 20, R is an alkyl group having a carbon number of 12 to 18.

8. The method of claim 1, wherein the reaction solution further contains a melting temperature adjusting agent.

9. The method of claim 1, wherein the oligonucleotide primers are substantially complementary to portions of the template nucleic acid fragment obtained in the step (i).

10. The method of claim 1, wherein only the 3'-terminal region of the oligonucleotide primers is substantially complementary to portions of the template nucleic acid fragment obtained in the step (i).

11. The method of claim 1, wherein at least one type of polymerase having strand displacement activity is a polymerase selected from the group consisting of *Bacillus stearothermophilus*-derived 5'→3' exonuclease-deficient Bst. DNA polymerase, *Bacillus caldotenax*-derived 5'→3'exonuclease-deficient Bca DNA polymerase, *Thermococcus litoralis*-derived 5'→3' exonuclease-deficient Vent. DNA polymerase, and *Alicyclobacillus acidocaldarius*-derived DNA polymerase.

12. The method of claim 1 wherein the reaction solution is incubated substantially isothermally at a temperature of 50°C or more.

13. The method of claim 1 wherein the time for the substantially isothermal incubation in the step (ii) is within 60 minutes.

14. A method for detecting a target RNA which comprises performing the method for amplification of nucleic acid of claim 1.

15. The method of claim 14 wherein the step (ii) comprises the following steps of:
(1) substantially isothermally incubating a reaction solution containing at least one type of deoxynucleotide triphosphate, at least one type of DNA polymerase having strand displacement activity, a divalent cation, at least one type of nonionic surfactant, at least two types of oligonucleotide primer containing a mutation site, and a nucleic acid fragment containing a reverse transcript of the target RNA as a template; and
(2) determining the presence or the absence of a mutation based on whether or not a nucleic acid amplification reaction takes place by a polymerase reaction that is initiated from the 3' end of the primer.


SEQUENCE LISTING

<110> FUJIFILM Corporation

<120> A method for detection of RNA

<130> FA8198A

<160> 2

<210> 1
<211> 20
<212> DNA
<213> Artificial Sequence

<220>
<223> Description of Artificial Sequence: Synthetic DNA

<400> 1
gggcatgggt cagaaggatt                    20

<210> 2
<211> 17
<212> DNA
<213> Artificial Sequence

<220>
<223> Description of Artificial Sequence: Synthetic DNA

<400> 2
cctcgtcgcc cacatag                    17

SEQUENCE LISTING
<110> FUJIFILM Corporation
<120> A method for detection of RNA
<130> FA8198A
<160> 2
<210> 1
<211> 20
<212> DNA
<213> Artificial Sequence
<220>
<223> Description of Artificial Sequence: Synthetic DNA
<400> 1
gggcatgggt cagaaggatt                    20
<210> 2
<211> 17
<212> DNA
<213> Artificial Sequence
<220>
<223> Description of Artificial Sequence: Synthetic DNA
<400> 2
cctcgtcgcc cacatag                    17